# EUROPÄISCHE PATENTSCHRIFT

(11) **EP 1 899 289 B1**
(45) Veröffentlichungstag und Bekanntmachung des Hinweises auf die Patenterteilung: **10.08.2011**
(21) Anmeldenummer: 06762044.3
(22) Anmeldetag: 14.06.2006
(51) Int. Cl.: C07C 41/16, C07C 43/275, C07F 9/50, C07C 209/10, C07C 211/56, C07C 211/58, C07C 255/58, C07D 295/02, C07C 281/02, C07C 253/30

(54) **VERFAHREN ZUR HERSTELLUNG VON ARYLAMINEN, ARYLETHERN UND ARYLTHIOETHERN**
METHOD FOR PRODUCING ARYL AMINES, ARYL ETHERS AND ARYL THIOETHERS
PROCEDE DE PRODUCTION D'ARYLAMINES, D'ARYLETHERS ET D'ARYLTHIOETHERS

(30) Priorität: 27.06.2005 DE 102005030400
(43) Veröffentlichungstag der Anmeldung: 19.03.2008
(73) Patentinhaber: Archimica GmbH, 65929 Frankfurt am Main (DE)
(72) Erfinder: MEUDT, Andreas, 65719 Hofheim (DE); NERDINGER, Sven, 83088 Kiefersfelden (DE); LEHNEMANN, Bernd, 60594 Frankfurt (DE); VOGEL, Till, 68219 Mannheim (DE); JUNG, Jörg, 65439 Flörsheim (DE); SNIECKUS, Victor, Kingston, Ontario K7L 2G4 (CA)
(74) Vertreter: Schweitzer, Klaus
(86) Internationale Anmeldenummer: PCT/EP2006/005719
(87) Internationale Veröffentlichungsnummer: WO 2007/000250

(56) Entgegenhaltungen:
- EP-A1- 0 667 350
- WO-A-00/68237
- WO-A1-01/21625
- WO-A2-2006/074315
- WÜLLNER ET AL: "Palladium-catalyzed amination of aromatic halides in water-containing solvent systems: a two-phase protocol" CHEM. COMMUN., 1998, Seiten 1509-1510, XP002402457
- HERD, OLIVER ET AL: "Water-Soluble Phosphines. 17. Novel Water-Soluble Secondary and Tertiary Phosphines with Disulfonated 1,1'-Biphenyl Backbones and Dibenzophosphole Moieties" INORGANIC CHEMISTRY , 41(20), 5034-5042 CODEN: INOCAJ; ISSN: 0020-1669, 2002, XP002402458
- HENSCHKE ET AL: "Synthesis and Applications of HexaPHEMP, a Novel Biaryl Diphosphine Ligand" ADV. SYNTH. CAT., Bd. 345, 2003, Seiten 300-307, XP002402459

## Beschreibung

Gemischte aryl- bzw. heteroarylsubstituierte Alkyl-/Arylamine sowie aryl- bzw. heteroarylsubstituierte Alkyl-/Arylether, vor allem mit funktionellen Gruppen in der Alkylkette, sind bedeutsame und äußerst vielseitig verwendbare Zwischenprodukte in der organischen Synthese. Die Bedeutung in der modernen organischen Synthese wird nur durch Limitierungen der Zugänglichkeit dieser Verbindungsklasse eingeschränkt. Standardverfahren zur Herstellung von gemischten aryl- bzw. heteroarylsubstituierten Alkyl-/Arylaminen sowie aryl- bzw. heteroarylsubstituierten Alkyl-/Arylethem ist die Ullmann-Reaktion, wobei die Reaktion sehr hohe Temperaturen benötigt, um vollständig abzulaufen. Jedoch tolerieren diese in der Regel drastischen Reaktionsbedingungen selten funktionelle Gruppen und reaktive Heteroaromaten und sind nicht oder nur sehr schlecht anwendbar auf elektronenarme Aromaten und zusätzlich nur schwierig zu kontrollieren. Modernere Verfahren zur Herstellung dieser Amine und Ether bedienen sich Pd- bzw. Ni-katalysierten Kupplungen von Aminen oder Alkoholen in Gegenwart verschiedener Liganden. Allerdings weisen die derzeit bekannten Verfahren allesamt noch verfahrenstechnische oder wirtschaftliche Nachteile auf, die die Anwendungsbreite erheblich einschränken. Unter anderem sind hier hohe Kosten der Katalysatoren/Liganden, hohe erforderliche Beladungen/Katalysatorkonzentrationen und schwierige Abtrennbarkeit des Katalysators vom Endprodukt zu erwähnen. Letzteres ist u. a. auch dadurch begründet, dass die bisher verwendeten Liganden alle weitgehend unpolar sind und sich dadurch bei wässrigen Aufarbeitungen bevorzugt in der organischen Phase aufhalten.

Es wäre sehr wünschenswert, ein Verfahren zu haben, das substituierte Alkyl- bzw. Arylamine, Alkohole bzw. Phenole und Halogenaromaten bzw. Halogenheteroaromaten in die entsprechenden gemischten aryl- bzw. heteroarylsubstituierten Alkyl-/Arylamine sowie aryl- bzw. heteroarylsubstituierten Alkyl-/Arylether überführen kann, dabei gleichzeitig sehr hohe Ausbeuten erzielt, mit sehr geringen Katalysatormengen auskommt und sich zusätzlich durch einfache Abtrennung des Liganden und des verwendeten Übergangsmetalls vom Produkt auszeichnet. Wie bereits erwähnt, lösen die bisher dafür veröffentlichten Syntheseverfahren dieses Problem nicht zufrieden stellend, wie anhand von einigen Beispielen weiter demonstriert werden soll:
- Verwendung teurer Liganden (z.B. P^{t}Bu₃, Hartwig et al., US6100398) sowie aufwendige Isolierung des Produktes durch Chromatographie.
- Verwendung von Liganden, die schwierig zu synthetisieren sind (Ferrocenbasierte Liganden, Hartwig et al., WO0211883), aufwendige Isolierung des Produktes durch Chromatographie.
- aufwendige bzw. schwierige, oft vielstufige Ligandensynthesen (Buchwald et al., WO 00/02887), aufwendige Isolierung des Produktes durch Chromatographie.

Weitere Methoden zur C, X-Bindungsknüpfung (X = O, N, S) aus Arylhalogeniden bzw. -sulfonaten unter Verwendung verschiedener Katalysatoren zeichnen sich durch folgende Nachteile aus (Wolfe, J. P.; Buchwald, S. L. J. Org. Chem. 2000, 65, 1444; , Wolfe, J. P.; Buchwald, S. L. J. Org. Chem. 2000, 65, 1158; Huang, J.; Grassa, G.; Nolan, S.P. Org. Lett. 1999, 1, 1307; Hartwig, J. F.; Kawatsura, M.; Hauck, S. I.; Shaughnessy, K. H.; Alcazar-Roman, L. M. J. Org. Chem. 1999, 64, 5575; Stauffer, S. I.; Hauck, S. I.; Lee, S.; Stambuli, J.; Hartwig, J. F. Org. Lett. 2000, 2, 1423):
- Die Reaktionstemperaturen sind in vielen Fällen sehr hoch, wodurch oft Nebenreaktionen und geringe Selektivitäten verursacht werden
- Für C,N-Bindungsknüpfungen gilt, dass die Selektivitäten für die Bildung der gewünschten Aniline im Gegensatz zu den ungewünschten Aminen bzw. Diarylaminen oft zu niedrig für eine wirtschaftliche Anwendung sind
- Die Abtrennung des Katalysators vom Produkt ist oft schwierig, da die gebildeten Amine die Übergangsmetalle recht effektiv binden, andererseits aber insbesondere für Pharma-Feinchemikalien sehr niedrige Spezifikationsgrenzen einzuhalten sind (z.B. < 10 oder < 5 ppm). Zusätzlich sind die üblicherweise verwendeten Katalysatorsysteme in verschiedenen anderen Reaktionen hochaktiv, so dass auch in Folgestufen unerwünschte Nebenreaktionen katalysiert werden können

In CHEM. COMMUN. 1998, 1509-1510 wird ein Kreuzkupplungsverfahren zur Herstellung von Aminen beschrieben, bei dem ein Arylphosphinligand verwendet wird, in dem die Phosphingruppe über eine Methylengruppe an die Arylgruppe gebunden ist.

In der nicht vorveröffentlichten WO 2006/074315 werden Phosphinliganden beschrieben, bei denen nur theoretisch die Möglichkeit besteht, dass eine Arylgruppe sowohl eine Sulfongruppe und eine OH-Gruppe trägt.

Das vorliegende Verfahren löst alle diese Probleme und betrifft ein Verfahren zur Herstellung von Aryl- und Heteroarylaminen, aryl- bzw. heteroarylsubstituierten Alkyl-/Arylethern oder aryl- bzw. heteroarylsubstituierten Alkyl-/Arylthioethem (III) durch Kreuzkupplung von primären oder sekundären Alkyl- bzw. Arylaminen, Alkoholen bzw. Phenolen oder Thioalkoholen bzw. Thioethern (II) mit substituierten Aryl- oder Heteroarylverbindungen (I), in Gegenwart einer Brønsted-Base und eines Katalysators oder Präkatalysators enthaltend
a.) ein Übergangsmetall, einen Komplex, Salz oder eine Verbindung dieses Übergangsmetalls aus der Gruppe {V, Mn, Fe, Co, Ni, Rh, Pd, Ir, Pt}, und
b.) mindestens einen sulfonierten Phosphanliganden
in einem Lösungsmittel oder Lösungsmittelgemisch entsprechend Schema 1.

Das erfindungsgemäße Verfahren zeichnet sich durch die folgenden Vorteile aus:
- Bei sehr geringen Katalysatorbeladungen werden hohe Ausbeuten und sehr hohen Selektivitäten erzielt
- Es bietet einen einfachen und wirtschaftlichen Zugang zu sulfonierten Liganden durch Sulfonierung kommerziell erhältlicher oder einfach zugänglicher Liganden (Beispiel: die entsprechend US 5789623 einfach und sehr wirtschaftlich zugänglichen 2-Hydroxy-2'-dialkylphosphinobiaryle können durch einfache Behandlung mit Schwefelsäure in die entsprechenden sulfonierten Liganden überführt werden. Durch die Zugänglichkeit/Erhältlichkeit der entsprechenden Oxaphosphorinchloride (z.B. 10-Chlor-10H-9-oxa-10-phosphaphenanthren) handelt es sich insgesamt um eine sehr einfache und mit guten Ausbeuten verlaufende Zweistufenreaktion, die sich durch sehr hohe Flexibilität auszeichnet, da verschiedenste Reste am Phosphor sehr einfach eingeführt werden können.)
- Die mit dem erfindungsgemäßen Verfahren erzielten Katalysatoraktivitäten sind sehr hoch, da der Ligand in der Reaktionsmischung als Anion vorliegt und dadurch besondere elektronische Effekte aufweist (vgl. hierzu insbesondere Beispiel 13).
- Ein Feintuning der elektronischen Eigenschaften der erfindungsgemäßen Liganden ist durch die Möglichkeit verschiedener Gegenionen (Metallkationen, substituierte Ammoniumsalze etc.) möglich. Insbesondere bei zweifach deprotonierbaren Liganden, wie z.B. bei sulfoniertem 2-Hydroxy-2'-dialkylphosphinobiphenylen, kann hier sehr gezielt auf die jeweiligen Erfordernisse einer bestimmten Reaktion maßgeschneidert werden.
- Einfache Abtrennung des Liganden sowie Metalls vom Produkt durch wässrige Extraktion, da durch die sehr hohe Acdität/Polarität der sulfonierten Liganden diese sich bevorzugt in der wässrigen Phase aufhalten
- Die Reaktion kann auch in protischen Lösungsmitteln wie z.B. substituierten Alkoholen durchgeführt werden, mit oft positivem Einfluss auf die Selektivität/Reaktivität
- Das erfindungsgemäße Verfahren erweitert durch die genannten, zusätzlich feinjustierbaren Parameter, die Anwendungsbreite der bisher bekannten C,X-Kupplungstechnologien außerordentlich
- Außergewöhnliche Aktivität der sulfonierten Liganden/Katalysatorsysteme (vgl. Beispiel 13), dadurch oft rasche Reaktionen und kurze Reaktionszeiten

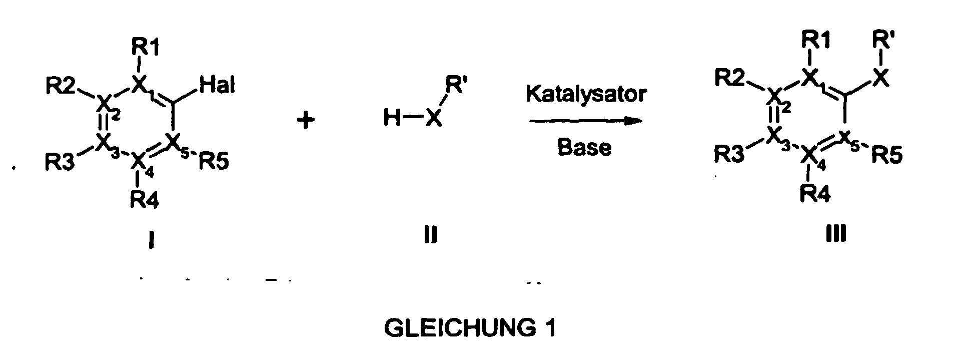

In Gleichung 1 steht Hal für Fluor, Chlor, Brom, lod, Alkoxy oder für Sulfonat-Abgangsgruppen wie beispielsweise Trifluormethansulfonat (Triflat), Nonafluortrimethylmethansulfonat (Nonaflat), Methansulfonat, Benzolsulfonat, para-Toluolsulfonat,

X steht für O, S oder NR",

X₁₋₅ bedeuten unabhängig voneinander Kohlenstoff oder XᵢRᵢ stehen für Stickstoff, oder jeweils zwei benachbarte über eine formale Doppelbindung verbundene XᵢRᵢ stehen gemeinsam für O (Furane), S (Thiophene), NH oder NRᵢ (Pyrrole).

Bevorzugte Verbindungen der Formel (I), die nach dem erfindungsgemäßen Verfahren umgesetzt werden können, sind z.B. Benzole, Pyridine, Pyrimidine, Pyrazine, Pyridazine, Furane, Thiophene, Pyrrole, beliebig N-substituierte Pyrrole oder Naphthaline, Chinoline, Indole, Benzofurane usw.

Die Reste R₁₋₅ stehen für Substituenten aus der Gruppe {Wasserstoff, Methyl, primäre, sekundäre oder tertiäre, cyclische oder acyclische Alkylreste mit 2 bis 20 C-Atomen, bei denen gegebenenfalls ein oder mehrere Wasserstoffatome durch Fluor oder Chlor oder Brom ersetzt sind, z.B. CF₃, substituierte cyclische oder acyclische Alkylgruppen, Hydroxy, Alkoxy, Amino, Alkylamino, Dialkylamino, Arylamino, Diarylamino, Alkylarylamino, Pentafluorsulfuranyl, Phenyl, substituiertes Phenyl, Heteroaryl, substituiertes Heteroaryl, Thio, Alkylthio, Arylthio, Diarylphosphino, Dialkylphosphino, Alkylarylphosphino, gegebenenfalls substituiertes Aminocarbonyl, CO₂⁻, Alkyl- oder Aryloxycarbonyl, Hydroxyalkyl, Alkoxyalkyl, Fluor oder Chlor, Nitro, Cyano, Aryl- oder Alkylsulfon, Aryl- oder Alkylsulfonyl}, oder es können jeweils zwei benachbarte Reste R₁₋₅ zusammen einem aromatischen, heteroaromatischen oder aliphatischen ankondensierten Ring entsprechen.

Für X = O oder S kann R' für gleiche oder unterschiedliche Reste aus der Gruppe {Wasserstoff, Methyl, lineares, verzweigtes C₁₋C₂₀ Alkyl oder cyclisches Alkyl, substituiertes oder unsubstituiertes Aryl- oder Heteroaryl} stehen.

Für X = NR" können R' und R" unabhängig voneinander für gleiche oder unterschiedliche Reste aus der Gruppe {Wasserstoff, Methyl, lineares, verzweigtes C₁-C₂₀ Alkyl oder cyclisches Alkyl, substituiertes oder unsubstituiertes Aryl- oder Heteroaryl} stehen oder zusammen einen Ring bilden.

Typische Beispiele für die Verbindung II sind damit Methyl-, Ethyl-, 1-Methylethyl-, Propyl-, 1-Methylpropyl-, 2-Methylpropyl-, 1,1-Dimethylethyl-, Butyl- und Pentylamin, Cyclopropyl-, Cyclobutyl-, Cyclopentyl-, Cyclohexylamin, Phenyl-, Benzylamin, Morpholin sowie tert-Butanol, Isopropanol, Neopentylalkohol oder n-Alkanole, Phenol oder Thiophenol.

Als Katalysator wird erfindungsgemäß ein Übergangsmetall, bevorzugt auf einem Träger wie z. B. Palladium auf Kohle, oder ein Salz, ein Komplex oder eine metallorganische Verbindung dieses Metalls ausgewählt aus der Gruppe {V, Mn, Fe, Co, Ni, Rh, Pd, Ir, Pt}, bevorzugt Palladium oder Nickel, mit einem sulfonierten Liganden verwendet. Der Katalysator kann in fertiger Form zugesetzt werden oder sich in situ bilden, z.B. aus einem Präkatalysator durch Reduktion oder Hydrolyse oder aus einem Metallsalz und zugesetztem Liganden durch Komplexbildung. Der Katalysator wird in Kombination mit einem oder mehreren, mindestens jedoch einem, sulfonierten phosphorhaltigen Liganden eingesetzt. Das Metall kann in beliebiger Oxidationsstufe eingesetzt werden. Erfindungsgemäß wird es im Verhältnis zum Reaktanden I in Mengen von 0.0001 Mol-% bis 100 Mol-%, bevorzugt zwischen 0.01 und 10 Mol-%, besonders bevorzugt zwischen 0.01 und 1 Mol-% eingesetzt.

Erfindungsgemäß werden sulfonierte Phosphanliganden verwendet, die sich dadurch auszeichnen, dass mindestens eine Sulfonsäuregruppe oder ein Salz einer Sulfonsäuregruppe im Molekül enthalten ist.

Es werden Liganden der nachstehend abgebildeten Struktur (IV) 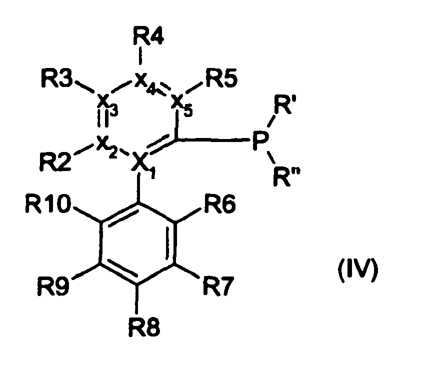 in Verbindung mit Übergangsmetallen, bevorzugt Palladium oder Nickel als Katalysator, verwendet.

Hierbei steht
X₁ für Kohlenstoff oder Stickstoff, X₂₋₅ unabhängig voneinander entweder für Kohlenstoff, XᵢRᵢ für Stickstoff oder jeweils zwei benachbarte über eine formale Doppelbindung verbundene XᵢRᵢ mit i=2,3,4,5 gemeinsam für O (Furane), S (Thiophene), NH oder NRᵢ (Pyrrole);
die Reste R₂₋₁₀, wobei mindestens ein Rest eine Sulfonsäure- oder Sulfonatgruppe enthält, stehen für Substituenten aus der Gruppe {Wasserstoff, Methyl, primäre, sekundäre oder tertiäre, cyclische oder acyclische Alkylreste mit 2 bis 20 C-Atomen, bei denen gegebenenfalls ein oder mehrere Wasserstoffatome durch Fluor oder Chlor oder Brom ersetzt sind, z.B. CF₃, substituierte cyclische oder acyclische Alkylgruppen, Hydroxy, Alkoxy, Amino, Alkylamino, Dialkylamino, Arylamino, Diarylamino, Alkylarylamino, Pentafluorsulfuranyl, Phenyl, substituiertes Phenyl, Heteroaryl, substituiertes Heteroaryl, Thio, Alkylthio, Arylthio, Diarylphosphino, Dialkylphosphino, Alkylarylphosphino, gegebenenfalls substituiertes Aminocarbonyl, CO₂⁻, Alkyl- oder Aryloxycarbonyl, Hydroxyalkyl, Alkoxyalkyl, Nitro, Cyano, Aryl- oder Alkylsulfon, Aryl- oder Alkylsulfonyl}, oder jeweils zwei benachbarte Reste R₂₋₅ stehen zusammen für einem aromatischen, heteroaromatischen oder aliphatischen ankondensierten Ring;
R' und R" bedeuten unabhängig voneinander gleiche oder unterschiedliche Reste aus der Gruppe {Wasserstoff, Methyl, lineares, verzweigtes oder cyclisches C₁₋C₂₀-Alkyl, gegebenenfalls substituiert, Phenyl, gegebenenfalls substituiert} oder bilden zusammen einen Ring und stehen für ein verbrückendes Strukturelement aus der Gruppe {gegebenenfalls substituiertes Alkylen, verzweigtes Alkylen, cyclisches Alkylen} oder unabhängig voneinander für einen oder zwei polycyclische Reste, wie z.B. Norbomyl oder Adamantyl .

Besonders bevorzugt sind hierbei solche Derivate, die neben mindestens einer Sulfonsäuregruppe auch eine weitere deprotonierbare Funktion im Molekül enthalten, wie z.B. eine freie OH-Gruppe im sulfonierten Ring.

Weiterhin betrifft die vorliegende Erfindung neuartige sulfonierte Liganden der Formeln (VII) und (VIII) der nachfolgend dargestellten Strukturen, die sich hervorragend für die Herstellung von Katalysatoren zur Anwendung in der organisch-chemischen Synthese eignen 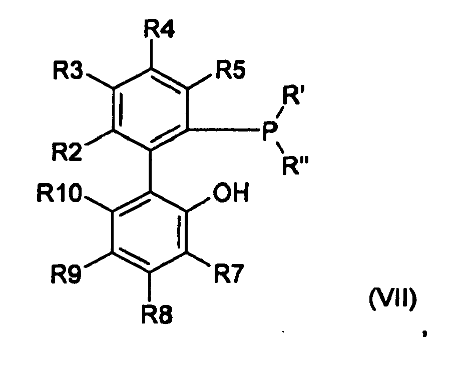 worin mindestens ein Rest Rᵢ eine Sulfonsäure- oder Sulfonatgruppe repräsentiert und die Reste R₂₋₅ und R₇₋₁₀ für Substituenten aus der Gruppe {Wasserstoff, Methyl, primäre, sekundäre oder tertiäre, cyclische oder acyclische Alkylreste mit 2 bis 20 C-Atomen, bei denen gegebenenfalls ein oder mehrere Wasserstoffatome durch Fluor oder Chlor oder Brom ersetzt sind, substituierte cyclische oder acyclische Alkylgruppen, Hydroxy, Alkoxy, Amino, Alkylamino, Dialkylamino, Arylamino, Diarylamino, Alkylarylamino, Phenyl, substituiertes Phenyl, Heteroaryl, substituiertes Heteroaryl, Thio, Alkylthio, Arylthio, Diarylphosphino, Dialkylphosphino, Alkylarylphosphino, gegebenenfalls substituiertes Aminocarbonyl, CO₂⁻, Alkyl- oder Aryloxycarbonyl, Hydroxyalkyl, Alkoxyalkyl, Nitro, Cyano, Aryl- oder Alkylsulfon, Aryl- oder Alkylsulfonyl} stehen oder jeweils zwei benachbarte Reste R₂₋₅ zusammen für einen aromatischen, heteroaromatischen oder aliphatischen ankondensierten Ring stehen,

R' und R" unabhängig voneinander für gleiche oder unterschiedliche Reste aus der Gruppe {Wasserstoff, Methyl, lineares, verzweigtes oder cyclisches Alkyl, Phenyl} stehen oder zusammen einen Ring bilden und für ein verbrückendes Strukturelement aus der Gruppe {Alkylen, verzweigtes Alkylen, cyclisches Alkylen} oder unabhängig voneinander für einen oder zwei polycyclische Reste, wie z.B. Norbornyl- oder Adamantyl-, stehen.

Ebenso umfasst die vorliegende Erfindung neuartige, sulfonierte Liganden der Struktur 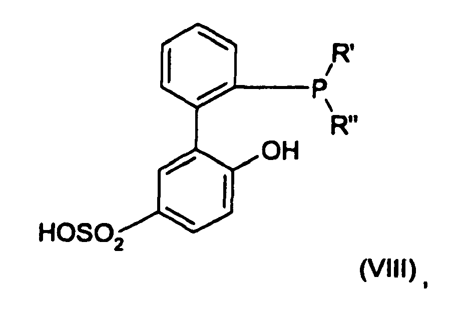 worin R' und R" unabhängig voneinander für gleiche oder unterschiedliche Reste aus der Gruppe {Wasserstoff, Methyl, lineares, verzweigtes oder cyclisches Alkyl, Phenyl} stehen oder zusammen einen Ring bilden und für ein verbrückendes Strukturelement aus der Gruppe {Alkylen, verzweigtes Alkylen, cyclisches Alkylen} oder unabhängig voneinander für einen oder zwei polycyclische Reste, wie z.B. Norbornyl- oder Adamantyl-, stehen.

Geeignete Katalysatoren oder Präkatalysatoren für das erfindungsgemäße Verfahren sind beispielsweise Komplexe von Palladium oder Nickel mit sulfonierten Biarylphosphanen, die z.T. sehr einfach und wirtschaftlich zugänglich sind (z.B. IV und V, zur Herstellung vgl. EP 0 795 559) oder als Vertreter des dritten beschriebenen Typs die kommerziell erhältlichen sulfonierten Triphenylphosphine TPPTS, TPPDS und TPPMS VI a-c (ABBILDUNG I).

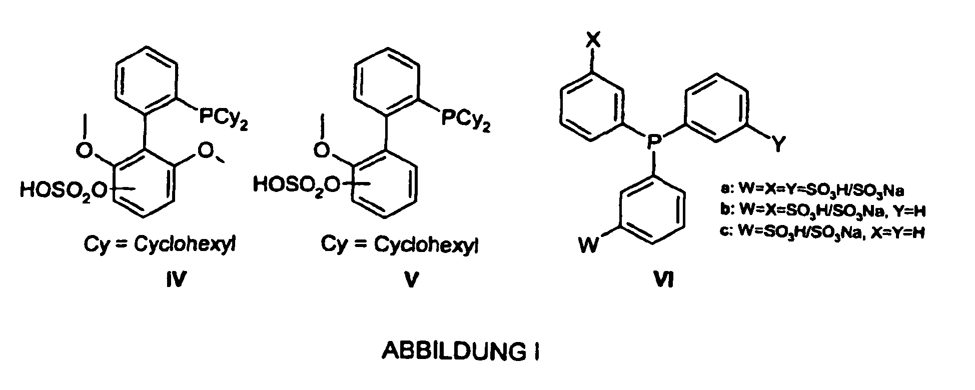

Der Zusatz von Bronsted-Basen zum Reaktionsgemisch ist notwendig, um akzeptable Reaktionsgeschwindigkeiten zu erzielen. Als Basen gut geeignet sind z.B. Hydroxide, Alkoholate und Fluoride der Alkali- und Erdalkalimetalle, Carbonate, Hydrogencarbonate und Phosphate der Alkalimetalle und ihre Gemische. Besonders geeignet sind die Basen der Gruppe {Kalium-tert-butylat, Natrium-tert-butylat, Cäsium-tert-butylat, Lithium-tert-butylat sowie die entsprechenden Isopropylate}. Dabei wird üblicherweise mindestens die Stoffmenge an Base eingesetzt, die der Stoffmenge des zu kuppelnden Amins, Phenols oder Alkohols II entspricht, zumeist werden 1,0 bis 6 Äquivalente, vorzugsweise 1,2 bis 3 Äquivalente an Base bezogen auf die Verbindung (II) eingesetzt.

Die Reaktion wird in einem geeigneten Lösungsmittel oder einem ein- oder mehrphasigen Lösungsmittelgemisch durchgeführt, das ein hinreichendes Lösevermögen für alle beteiligten Reaktanden hat, wobei auch heterogene Durchführungen möglich sind (z.B. Einsatz fast unlöslicher Basen). Vorzugsweise wird die Reaktion in polaren, aprotischen oder protischen Lösungsmitteln durchgeführt. Gut geeignet sind offenkettige und cyclische Ether und Diether, Oligo- und Polyether sowie substituierte einfache oder mehrfache Alkohole und gegebenenfalls substituierte Aromaten. Besonders bevorzugt werden ein oder Gemische mehrerer Lösungsmittel der Gruppe {Diglyme, substituierte Glymes, 1,4-Dioxan, Isopropanol, tert-Butanol, 2,2-Dimethyl-1-propanol, Toluol, Xylol} eingesetzt.

Die Reaktion kann bei Temperaturen zwischen Raumtemperatur und dem Siedepunkt des verwendeten Lösungsmittels beim verwendeten Druck durchgeführt werden. Um eine schnellere Reaktion zu erzielen, ist die Durchführung bei erhöhten Temperaturen im Bereich von 0 bis 240°C bevorzugt. Besonders bevorzugt ist der Temperaturbereich von 20 bis 200°C, insbesondere von 50 bis 150°C.

Die Konzentration der Reaktanden kann in weiten Bereichen variiert werden. Zweckmäßigerweise wird die Reaktion in einer möglichst hohen Konzentration durchgeführt, wobei die Löslichkeiten der Reaktionspartner und Reagenzien im jeweiligen Reaktionsmedium beachtet werden müssen. Bevorzugt wird die Reaktion im Bereich zwischen 0.05 und 5 mol/l bezogen auf den im Unterschuss vorliegenden Reaktanden durchgeführt (abhängig von den relativen Preisen der Reaktanden).

Amin, Alkohol, Phenol, Thioalkohol oder Thiophenol der Formel (II) und aromatischer bzw. heteroaromatischer Reaktionspartner (I) können in Molverhältnissen von 10:1 bis 1:10 eingesetzt werden, bevorzugt sind Verhältnisse von 3:1 bis 1:3 und besonders bevorzugt sind Verhältnisse von 1,2:1 bis 1:1,2.

In einer der bevorzugten Ausführungsformen werden alle Materialien vorgelegt und das Gemisch unter Rühren auf Reaktionstemperatur erhitzt. In einer weiteren bevorzugten Ausführungsform, die sich besonders für die Anwendung im großen Maßstab eignet, wird die Verbindung (II) und gegebenenfalls weitere Reaktanden, z.B. Base und Katalysator oder Präkatalysator während der Reaktion zum Reaktionsgemisch dosiert. Alternativ kann die Reaktion auch durch langsame Zugabe der Base dosiert-kontrolliert durchgeführt werden.

Die Aufarbeitung erfolgt üblicherweise mit einem Gemisch aus aromatischen Kohlenwasserstoffen/Wasser unter Abtrennung der wässrigen Phase, die die anorganischen Bestandteile sowie Ligand und Übergangsmetall aufnimmt, wobei das Produkt in der organischen Phase verbleibt, wenn nicht vorhandene saure Funktionsgruppen zu einem abweichenden Phasenverhalten führen. Gegebenenfalls können ionische Flüssigkeiten zur Abtrennung der polareren Bestandteile eingesetzt werden. Das Produkt wird bevorzugt durch Fällung oder Destillation aus der organischen Phase isoliert, z.B. durch Einengen oder durch Zusatz von Fällungsmitteln. Zumeist ist eine zusätzliche Reinigung oder nachträgliche Abtrennung von Übergangsmetall oder Ligand z.B. durch Umkristallisation oder Chromatographie unnötig. Die isolierten Ausbeuten liegen meistens im Bereich von 60 bis 100 %, vorzugsweise im Bereich > 75 bis 100, insbesondere > 80 bis 100. Die Selektivitäten sind dabei erfindungsgemäß sehr hoch, es lassen sich meist Bedingungen finden, unter denen bis auf sehr geringe Mengen Dehalogenierungsprodukt keine weiteren Nebenprodukte nachweisbar sind.

Das erfindungsgemäße Verfahren eröffnet eine vor allem in der Aufarbeitung und Abtrennung von Katalysator/Liganden sehr ökonomische Methode, um gemischte Aryl- und Heteroarylamine sowie aryl- bzw. heteroarylsubstituierte Alkyl-/Arylether bzw. Thioether ausgehend von den entsprechenden primären oder sekundären Alkyl- bzw. Arylaminen, Alkoholen bzw. Phenolen, Thioalkoholen bzw. Thiophenolen oder ihren Derivaten und den entsprechenden Aryl- oder Heteroarylhalogeniden oder -sulfonaten herzustellen und liefert die Produkte im allgemeinen in sehr hohen Reinheiten ohne aufwendige Reinigungsprozeduren.

Das erfindungsgemäße Verfahren soll durch die nachfolgenden Beispiele erläutert werden, ohne die Erfindung darauf zu beschränken:

### Beispiel 1: Herstellung des Liganden 2'-Hydroxy-2-dicyclohexylphosphino-biphenyl-4'-sulfonsäure (HBPNS)

1,099 g (3,0 mmol) 2-Hydroxy-2'-diphenylphosphinobiphenyl wurden unter Schutzgasatmosphäre in einem Eisbad vorgekühlt. Anschließend wurden 2.0 ml konzentrierte Schwefelsäure aus einer Spritze langsam zudosiert. Nach Aufwärmen auf Raumtemperatur wurde die gebildete Suspension für weitere ca. 2 Stunden gerührt, bis sich aller Feststoff gelöst hatte. Man erhielt eine homogene, viskose und leicht bräunlich gefärbte Suspension.

Die Reaktionsmischung wurde erneut in einem Eisbad gekühlt und anschließend mit Eis gequencht. Mit konzentrierter Natronlauge wurde der gebildete Niederschlag vollständig aufgelöst. Nach Verdünnung mit 75 ml Wasser und Ansäuern mit 1 N Schwefelsäure wurde der Niederschlag abfiltriert und so lange mit Wasser gewaschen, bis das ablaufende Waschwasser neutralen pH-Wert zeigt. Der weiße Filterkuchen wurde noch einmal mit Methanol gewaschen und im Vakuum getrocknet. Man erhielt 1.093 g (2,45 mmol, 82 %) 2-Hydroxy-2'-diphenylphosphinobiphenyl-5-sulfonsäure als weiße Kristalle.

Spektroskopische Daten

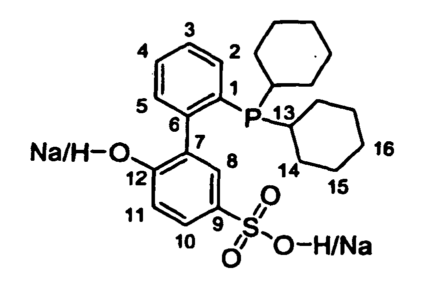

Schmelzpunkt (freie Säure):
285 - 295°C (Zersetzung).
¹H-NMR (D₂O/NaOH) (Natrium-Salz):
δ/ppm = 0.903 - 1.201 (m, 10H, 5xCH₂); 1.439 - 1.726 (m, 10H, 5xCH₂); 1.782 - 1.852 (m, 2H, 2xCH); 6.526 (d, J = 8.16Hz, 11-CH); 7.210 - 7.300 (m, 3H, 3,4,8-CH); 7.344
(d, J = 5.95 Hz, 1H, 2-CH); 7.418 (d, J = 7.67Hz, 1H, 10-CH); 7.547 (d, J = 5.25 Hz, 1H, 5-CH).
¹³C-NMR (D₂O/NaOH) (Natrium-Salz):
δ/ppm = 26.0, 26.1, 26.7, 26.8, 26.9, 27.0, 27.1, 27.2, 29.2, 29.3, 29.5, 29.8, 30.1 and 30.3 (14, 15, 16-CH₂); 33.2 (d, J = 8Hz) und 34.2 (d, J = 9Hz, 13,13'-CH); 119.0 (11-C); 125.0 (9-C); 126.3 (4-CH); 128.6 (2-CH); 129.6 (3-CH); 131.5 (d, J = 5Hz, 8-CH); 132.0 (d, J = 7Hz, 1-C); 132.7 (5-CH); 134.3 (d, J = 9Hz, 6-C); 148.6 (d, J = 27Hz, 7-C); 168.3 (12-C).
³¹P-NMR (D₂O/NaOH) (Natrium-Salz):
δ = -10.6 ppm.

HRMS (C₂₄H₃₁O₄PS) (freie Säure):
berechnet: 485.1318 (M+K)
gefunden: 485.1314 (M+K)

IR (KBr) (freie Säure):
v/cm⁻¹ = 3445, 3062, 2946, 2857, 1604, 1415, 1233, 1168, 1112, 1029, 1012, 832, 675, 593.

UVNIS (NaOH, 1N, c = 1*10⁻⁴ M):
λ (max) = 302 nm (e: 4032)
ε (max) = 18670 (λ: 224 nm)

### Beispiel 2: Kupplung von 2-Brom-4-fluortoluol mit 2,3-Dimethylanilin zu 5-Fluor-2,2',3'-trimethyl-diphenylamin

189 mg (1 mmol) 2-Brom-4-fluortoluol, 121 mg (1 mmol) 2,3-Dimethylanilin,
192 mg (2 mmol) Natrium-tert-butoxid, 4,4 mg Palladium(II)-acetat (2 Mol-%) und
26,8 mg des HBPNS-Liganden (6 Mol-%) wurden in 6 ml entgastem, wasserfreien Diglyme für 15 h auf 120°C erhitzt. Nach dem Abkühlen wurde das Reaktionsgemisch auf 10 ml Wasser gegeben und das Gemisch mit 10 ml Toluol extrahiert. Die Toluolphase wurde zur Entfernung von Diglyme-Resten mit 5 ml Wasser gewaschen und am Rotationsverdampfer eingeengt. Nach Trocknung im Vakuum erhielt man 207 mg (0.90 mmol, 90 %) des Produkts.

### Beispiel 3: Kupplung von 1-Bromnaphthalin mit 2,3-Dimethylanilin zu (2,3-Dimethylphenyl)-naphthalin-1-ylamin

Der Versuch wurde wie zuvor beschrieben durchgeführt, allerdings wurden anstelle von 2-Brom-4-fluortoluol 207 mg 1-Bromnaphthalin (1 mmol) und anstelle von Palladium(II)-acetat 14 mg Tris(dibenzylidenaceton)dipalladium(0) (1,5 mmol, 3 Mol-% Pd) eingesetzt. Die Ausbeute betrug 210 mg (0.85 mmol, 85%).

### Beispiel 4: Kupplung von 1-Bromnaphthalin mit 4-Aminobenzonitril zu (4-Cyanophenyl)-naphthalin-1-ylamin

Der Versuch wurde wie zuvor beschrieben durchgeführt, allerdings wurde anstelle des Dimethylanilins 118 mg 4-Aminobenzonitril (1 mmol) eingesetzt. Die Katalysatormenge wurde verringert auf 2,2 mg Palladium(II)-acetat (1 mol-%) und die Ligandmenge wurde verringert auf 8,0 mg (1,8 mol-%). Man erhielt eine Ausbeute von 181 mg (0,74 mmol, 74 %).

### Beispiel 5: Kupplung von 3-Brom-4-fluortoluol mit 4-Aminobenzonitril zu 4'-Cyano-2-fluor-5-methyldiphenylamin

189 mg (1 mmol) 3-Brom-4-fluortoluol, 118 mg 4-Aminobenzonitril (1 mmol),
192 mg (2 mmol) Natrium-tert-butoxid, 2.2 mg Palladium(II)-acetat (1 mol-%) und 4,5 mg des HBPNS-Liganden (1 Mol-%) wurden in 6 ml entgastem tert-Butanol für 30 h zum Rückfluss erhitzt. Nach dem Abkühlen wurde das Reaktionsgemisch auf 10 ml Wasser gegeben und das Gemisch mit 10 ml Toluol extrahiert. Die Toluolphase wurde zur Entfernung von tert-Butanol-Resten mit 5 ml Wasser gewaschen und am Rotationsverdampfer eingeengt. Nach Trocknung im Vakuum erhielt man 178 mg (0,79 mmol, 79 %) des Produkts.

### Beispiel 6: Kupplung von 1-Chlomaphthalin mit 4-Aminobenzonitril zu (4-Cyanophenyl)-naphthalin-1-ylamin

118 mg 4-Aminobenzonitril (1 mmol), 163 mg 1-Chlomaphthalin (1 mmol), 192 mg (2 mmol) Natrium-tert-butoxid, 2.2 mg Palladium(II)-acetat (1 mol-%) und 4,5 mg des HBPNS-Liganden (1 mol-%) wurden in 6 ml entgastem Diglyme für 15 h auf 120°C erhitzt. Nach dem Abkühlen wurde das Reaktionsgemisch auf 10 ml Wasser gegeben und das Gemisch mit 10 ml Toluol extrahiert. Die Toluolphase wurde zur Entfernung von Lösemittelresten mit 5 ml Wasser gewaschen und am Rotationsverdampfer eingeengt. Nach Trocknung im Vakuum erhielt man 215 mg (0,89 mmol, 89 %) des Produkts.

### Beispiel 7: Kupplung von 1-Chlornaphthalin mit 4-Aminobenzonitril zu (4-Cyanophenyl)-naphthalin-1-ylamin

Die Kupplung wurde wie zuvor beschrieben durchgeführt, als Lösungsmittel wurde jedoch tert.-Butanol anstelle von Diglyme eingesetzt. Die Ausbeute betrug nach 30stündiger Reaktion bei Rückflusstemperatur 201 mg (0.82 mmol, 82 %).

### Beispiel 8: Kupplung von 1-Bromnaphthalin mit Morpholin zu 4-Naphthalin-1-ylmorpholin

87 mg wasserfreies Morpholin (1 mmol), 207 mg 1-Bromnaphthalin (1 mmol), 192 mg (2 mmol) Natrium-tert-butoxid, 2.2 mg Palladium(II)-acetat (1 mol-%) und 4,5 mg des HBPNS-Liganden (1 mol-%) wurden in 6 ml entgastem Diglyme für 24 h auf 120°C erhitzt. Nach dem Abkühlen wurde das Reaktionsgemisch auf 10 ml Wasser gegeben und das Gemisch mit 10 ml Toluol extrahiert. Die Toluolphase wurde zur Entfernung von Lösemittelresten mit 5 ml Wasser gewaschen und am Rotationsverdampfer eingeengt. Nach Trocknung im Vakuum erhielt man 156 mg (0.73 mmol, 73 %) des Produkts.

### Beispiel 9: Kupplung von 1-Chlornaphthalin mit Morpholin zu 4-Naphthalin-1-ylmorpholin

Die Reaktion wurde wie im vorangegangenen Beispiel beschrieben ausgeführt, das Bromnaphthalin wurde durch 163 mg 1-Chlomaphthalin (1 mmol) ersetzt. Man erhielt 147 mg (0,69 mmol, 69 %) des Produkts.

### Beispiel 10: Kupplung von 1-Bromnaphthalin mit tert-Butylcarbazat zu N'-Naphthalin-1-ylhydrazincarbonsäure-tert-butylester

Die Reaktion wurde wie Beispiel 8 durchgeführt, anstelle von Morpholin wurden 132 mg tert-Butylcarbazat (1 mmol) eingesetzt. Die Ausbeute an Produkt betrug 201 mg, (0,8 mmol, 78 %).

### Beispiel 11: Kupplung von 4-Bromtoluol mit 4-Methoxyphenol zu 1-Methoxy-4-(4-methylphenoxy)-benzol

124 mg 4-Methoxyphenol (1 mmol), 171 mg 4-Bromtoluol (1 mmol), 192 mg (2 mmol) Natrium-tert-butoxid, 2,2 mg Palladium(II)-acetat (1 mol-%) und 4,5 mg des HBPNS-Liganden (1 mol-%) wurden in 6 ml entgastem Diglyme für 24 h auf 120°C erhitzt. Nach dem Abkühlen wurde das Reaktionsgemisch auf 10 ml Wasser gegeben und das Gemisch mit 10 ml Toluol extrahiert. Die Toluolphase wurde zur Entfernung von Lösemittelresten mit 5 ml Wasser gewaschen und am Rotationsverdampfer eingeengt. Nach Trocknung im Vakuum erhielt man eine Ausbeute von 141 mg (0,66 mmol, 66 %).

### Beispiel 12: Kupplung von 2-Bromtoluol mit 4-Methoxyphenol zu 1-Methoxy-4-(2-methylphenoxy)-benzol

124 mg 4-Methoxyphenol (1 mmol), 171 mg 2-Bromtoluol (1 mmol), 192 mg (2 mmol) Natrium-tert-butoxid, 2,2 mg Palladium(II)-acetat (1 mol-%) und 4,5 mg des HBPNS-Liganden (1 mol-%) wurden in 6 ml entgastem Diglyme für 24 h auf 120°C erhitzt. Nach dem Abkühlen wurde das Reaktionsgemisch auf 10 ml Wasser gegeben und das Gemisch mit 10 ml Toluol extrahiert. Die Toluolphase wurde zur Entfernung von Lösemittelresten mit 5 ml Wasser gewaschen und am Rotationsverdampfer eingeengt. Nach Trocknung im Vakuum erhielt man eine Ausbeute von 133 mg (0,62 mmol, 62 %).

### Beispiel 13: Vergleich der Aktivität von sulfoniertem und unsulfoniertem 2-Hydroxy-2'-dicyclohexylphosphinobiphenyl

207 mg 1-Bromnaphthalin (1 mmol), 118 mg 4-Aminobenzonitril (1 mmol), 192 mg (2 mmol) Natrium-tert-butoxid, 2.2 mg Palladium(II)-acetat (1 mol-%) und 4,5 mg des HBPNS-Liganden (1 mol-%) wurden in 6 ml entgastem Diglyme für 29 h auf 120°C erhitzt. Parallel dazu wurde ein gleichartiger Versuch durchgeführt, bei dem der unsulfonierte Ligand 2'-Hydroxy-2-dicyclohexylphosphinobiphenyl (3,7 mg, 1 mol-%) eingesetzt wurde. In regelmäßigen Abständen (s. Tabelle) wurden Proben beider Reaktionen genommen und per GC analysiert:

| Zeit/min | Umsatz (sulfonierter Ligand) | Umsatz (unsulfonierter Ligand) |
|---|---|---|
| 180 | 18 % | 7 % |
| 240 | 29 % | 12 % |
| 300 | 48 % | 18 % |
| 540 | 71 % | 34 % |
| 620 | 89 % | 48 % |
| 1380 | 93 % | 79 % |

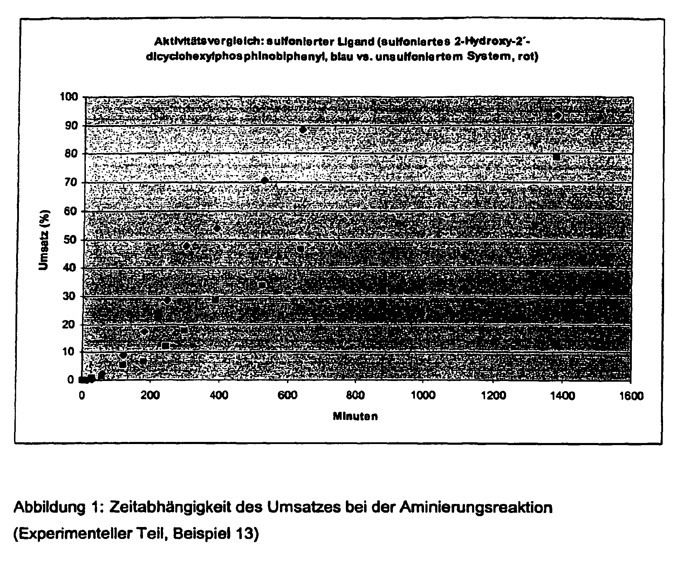

Nach 29 h wurde die Reaktion abgebrochen. Die isolierten Ausbeuten waren beim sulfonierten Liganden deutlich höher, vor allem aber ist die Reaktion im Falle des sulfonierten Liganden viel schneller (siehe Abbildung 1).

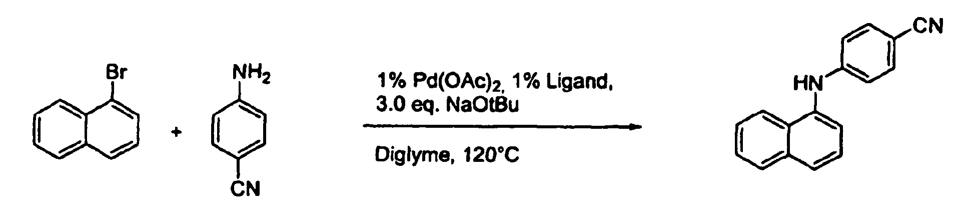

Man erkennt deutlich die geringere Reaktionsgeschwindigkeit im Fall des "klassischen" Liganden. So hat man nach 635 min (entspricht 5 h 35 min) mit dem sulfonierten Liganden schon fast 90 % Umsatz erreicht, mit dem unsulfonierten Liganden noch nicht einmal 50 %. Erst nach sehr langer Reaktionszeit nähern sich die Umsätze langsam an.

## Patentansprüche

1. Verfahren zur Herstellung von Aryl- oder Heteroarylaminen, -ethern, oder - thioethern (III) durch Kreuzkupplung von primären oder sekundären Aminen, Alkoholen oder Thioalkoholen (II) mit substituierten Aryl- oder Heteroarylverbindungen (I), in Gegenwart einer Brønsted-Base und eines Katalysators oder Präkatalysators enthaltend
a.) ein Übergangsmetall, einen Komplex, ein Salz oder eine Verbindung dieses Übergangsmetalls aus der Gruppe {V, Mn, Fe, Co, Ni, Rh, Pd, Ir, Pt}, und
b.) mindestens einen sulfonierten Phosphanliganden
in einem Lösungsmittel oder Lösungsmittelgemisch entsprechend Schema 1, 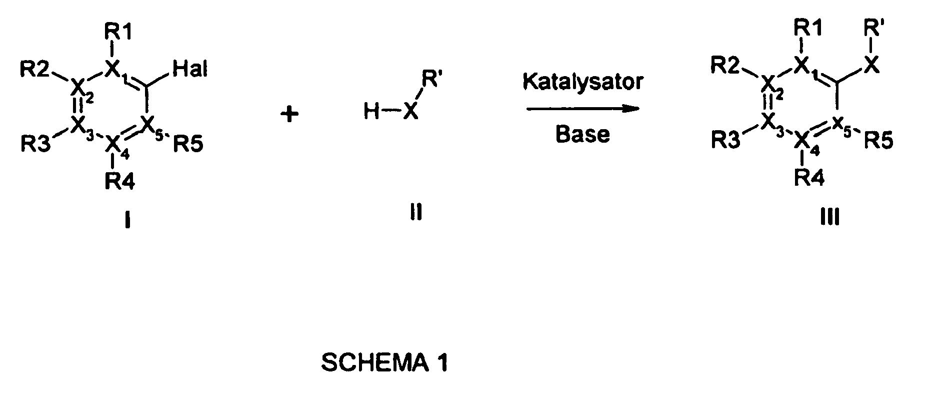
wobei Hal für Fluor, Chlor, Brom, Iod, Alkoxy, Trifluormethansulfonat, Nonafluortrimethyl-methansulfonat, Methansulfonat, 4-Toluolsulfonat, Benzolsulfonat, 2-Naphthalinsulfonat, 3-Nitrobenzolsulfonat, 4-Nitrobenzolsulfonat, 4-Chlorbenzolsulfonat, 2,4,6-Triisopro-pylbenzolsulfonat oder einem beliebigen anderen Sulfonat steht,
X steht für O, S oder NR",
X₁₋₅ unabhängig voneinander entweder für Kohlenstoff stehen, XᵢRᵢ für Stickstoff steht, oder jeweils zwei benachbarte über eine formale Doppelbindung verbundene XᵢRᵢ gemeinsam für O (Furane), S (Thiophene), NH oder NRᵢ (Pyrrole) stehen,
die Reste R₁₋₅ für Substituenten aus der Gruppe {Wasserstoff, Methyl, primäre, sekundäre oder tertiäre, cyclische oder acyclische Alkylreste mit 2 bis 20 C-Atomen, bei denen gegebenenfalls ein oder mehrere Wasserstoffatome durch Fluor oder Chlor oder Brom ersetzt sind, substituierte cyclische oder acyclische Alkylgruppen, Hydroxy, Alkoxy, Amino, Alkylamino, Dialkylamino, Arylamino, Diarylamino, Alkylarylamino, Pentafluorsulfuranyl, Phenyl, substituiertes Phenyl, Heteroaryl, substituiertes Heteroaryl, Thio, Alkylthio, Arylthio, Diarylphosphino, Dialkylphosphino, Alkylarylphosphino, gegebenenfalls substituiertes Aminocarbonyl, CO₂⁻, Alkyl- oder Aryloxycarbonyl, Hydroxyalkyl, Alkoxyalkyl, Fluor oder Chlor, Nitro, Cyano, Aryl- oder Alkylsulfon, Aryl- oder Alkylsulfonyl} stehen oder jeweils zwei benachbarte Reste R₁₋₅ zusammen für einem aromatischen, heteroaromatischen oder aliphatischen ankondensierten Ring stehen,
für X = O oder S kann R' für gleiche oder unterschiedliche Reste aus der Gruppe {Wasserstoff, Methyl, lineares, verzweigtes C₁-C₂₀ Alkyl oder cyclisches Alkyl, substituiertes oder unsubstituiertes Aryl- oder Heteroaryl} stehen,
oder X für NR" steht, wobei R' und R" unabhängig voneinander für gleiche oder unterschiedliche Reste aus der Gruppe {Wasserstoff, Methyl, lineares, verzweigtes C₁-C₂₀-Alkyl oder cyclisches Alkyl, substituiertes oder unsubstituiertes Aryl oder Heteroaryl} stehen oder zusammen einen Ring bilden,
und wobei ein sulfonierter Phosphanligand der Struktur 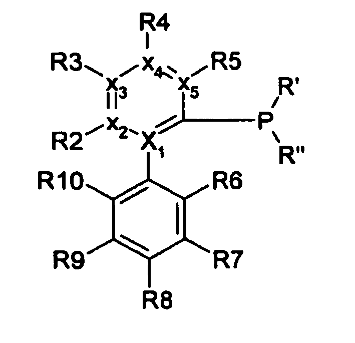 verwendet wird,
wobei
X₁ für Kohlenstoff oder Stickstoff steht,
X₂₋₅ unabhängig voneinander entweder für Kohlenstoff steht,
XᵢRᵢ für Stickstoff steht, oder jeweils zwei benachbarte über eine formale Doppelbindung verbundene XᵢRᵢ mit i = 2,3,4,5 gemeinsam für O (Furane), S (Thiophene), NH oder NRᵢ (Pyrrole) steht,
die Reste
R₂₋₁₀ stehen für Substituenten aus der Gruppe {Wasserstoff, Methyl, primäre, sekundäre oder tertiäre, cyclische oder acyclische Alkylreste mit 2 bis 20 C-Atomen, bei denen gegebenenfalls ein oder mehrere Wasserstoffatome durch Fluor oder Chlor oder Brom ersetzt sind, substituierte cyclische oder acyclische Alkylgruppen, Hydroxy, Alkoxy, Amino, Alkylamino, Dialkylamino, Arylamino, Diarylamino, Alkylarylamino, Phenyl, substituiertes Phenyl, Heteroaryl, substituiertes Heteroaryl, Thio, Alkylthio, Arylthio, Diarylphosphino, Dialkylphosphino, Alkylarylphosphino, gegebenenfalls substituiertes Aminocarbonyl, CO₂⁻, Alkyl- oder Aryloxycarbonyl, Hydroxyalkyl, Alkoxyalkyl, Nitro, Cyano, Aryl- oder Alkylsulfon, Aryl- oder Alkylsulfonyl},
wobei mindestens ein Rest eine Sulfonsäure- oder Sulfonatgruppe repräsentiert,
und wobei mindestens ein Rest in dem sulfonierten Ring eine freie OH-Gruppe ist
oder jeweils zwei benachbarte Reste
R₂₋₅ zusammen für einem aromatischen, heteroaromatischen oder aliphatischen ankondensierten Ring stehen,
R' und R" unabhängig voneinander für gleiche oder unterschiedliche Reste aus der Gruppe {Wasserstoff, Methyl, lineares, verzweigtes oder cyclisches Alkyl, Phenyl} stehen oder zusammen einen Ring bilden und für ein verbrückendes Strukturelement aus der Gruppe {Alkylen, verzweigtes Alkylen, cyclisches Alkylen} oder unabhängig voneinander für einen oder zwei polycyclische Reste, wie z.B. Norbornyl- oder Adamantyl-, stehen.

2. Verfahren nach Anspruch 1, **dadurch gekennzeichnet, dass** sulfonierte Phosphanliganden verwendet werden, die mindestens eine Sulfonsäuregruppe oder ein Metallsulfonat enthalten.

3. Verfahren nach Anspruch 1 und/oder 2, **dadurch gekennzeichnet dass** als Brønsted-Base ein Hydroxid, Alkoholat oder Amid der Alkali- oder Erdalkalimetalle oder ein Alkalicarbonat oder -phosphat oder Gemische dieser Verbindungen eingesetzt werden.

4. Verfahren nach mindestens einem der vorhergehenden Ansprüche, **dadurch gekennzeichnet, dass** 1,0 bis 3 Äquivalente an Base bezogen auf das Aryl- oder Heteroarylhalogenid bzw. Aryl- oder Heteroarylsulfonat eingesetzt werden.

5. Verfahren nach mindestens einem der vorhergehenden Ansprüche **dadurch gekennzeichnet, dass** als Lösungsmittel Kohlenwasserstoffe, halogenierte Kohlenwasserstoffe, offenkettige und cyclische Ether und Diether, Oligo- und Polyether, tertiäre Amine, DMSO, NMP, DMF, DMAc sowie substituierte einfache oder mehrfache Alkohole und gegebenenfalls substituierte Aromaten oder ein Gemisch mehrerer dieser Lösungsmittel eingesetzt wird.

6. Verfahren nach mindestens einem der vorhergehenden Ansprüche, **dadurch gekennzeichnet, dass** die Reaktion bei einer Temperatur im Bereich von 0 bis 240°C durchgeführt wird.

7. Verfahren nach mindestens einem der vorhergehenden Ansprüche, **dadurch gekennzeichnet, dass** der Katalysator im Verhältnis zum Reaktanden (I) in Mengen von 0.001 Mol-% bis 100 Mol-% eingesetzt wird.

8. Sulfonierte Liganden der Struktur 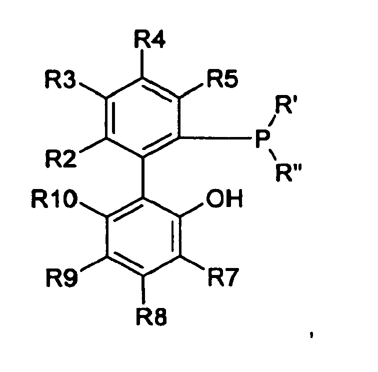 wobei die Reste
R₂₋₅und R₇₋₁₀ für Substituenten aus der Gruppe {Wasserstoff, Methyl, primäre, sekundäre oder tertiäre, cyclische oder acyclische Alkylreste mit 2 bis 20 C-Atomen, bei denen gegebenenfalls ein oder mehrere Wasserstoffatome durch Fluor oder Chlor oder Brom ersetzt sind, substituierte cyclische oder acyclische Alkylgruppen, Hydroxy, Alkoxy, Amino, Alkylamino, Dialkylamino, Arylamino, Diarylamino, Alkylarylamino, Phenyl, substituiertes Phenyl, Heteroaryl, substituiertes Heteroaryl, Thio, Alkylthio, Arylthio, Diarylphosphino, Dialkylphosphino, Alkylarylphosphino, gegebenenfalls substituiertes Aminocarbonyl, CO₂⁻, Alkyl- oder Aryloxycarbonyl, Hydroxyalkyl, Alkoxyalkyl, Nitro, Cyano, Aryl- oder Alkylsulfon, Aryl- oder Alkylsulfonyl}stehen ,
wobei mindestens ein Rest eine Sulfonsäure- oder Sulfonatgruppe repräsentiert,
oder jeweils zwei benachbarte Reste
R₂₋₅ zusammen für einem aromatischen, heteroaromatischen oder aliphatischen ankondensierten Ring stehen,
R' und R" unabhängig voneinander für gleiche oder unterschiedliche Reste aus der Gruppe {Wasserstoff, Methyl, lineares, verzweigtes oder cyclisches Alkyl, Phenyl} stehen oder zusammen einen Ring bilden und für ein verbrückendes Strukturelement aus der Gruppe {Alkylen, verzweigtes Alkylen, cyclisches Alkylen} oder unabhängig voneinander für einen oder zwei polycyclische Reste, wie z.B. Norbornyl- oder Adamantyl-, stehen.

9. Sulfonierte Liganden der Struktur 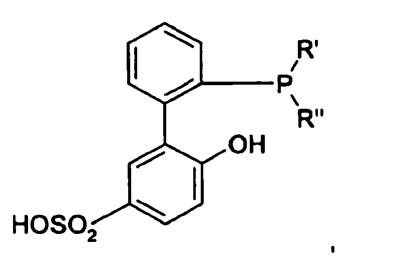 worin
R' und R" unabhängig voneinander für gleiche oder unterschiedliche Reste aus der Gruppe {Wasserstoff, Methyl, lineares, verzweigtes oder cyclisches Alkyl, Phenyl} stehen oder zusammen einen Ring bilden und für ein verbrückendes Strukturelement aus der Gruppe {Alkylen, verzweigtes Alkylen, cyclisches Alkylen} oder unabhängig voneinander für einen oder zwei polycyclische Reste, wie z.B. Norbornyl- oder Adamantyl-, stehen.

10. Komplexe, Mischungen, Salze oder Formulierungen enthaltend mindestens einen Liganden gemäß der Ansprüche 8 oder 9 und mindestens ein Metall, -komplex, -salz oder eine -verbindung der Gruppe {V, Mn, Fe, Co, Ni, Rh, Pd, Ir, Pt}.

11. Verwendung von Liganden gemäß den Ansprüchen 8 oder 9 für Katalysatoren in der organisch-chemischen Katalyse.

12. Verfahren zur Herstellung von sulfonierten Liganden gemäß Anspruch 8 oder 9 aus 2-Hydroxy-2'-phosphinobiphenylen durch elektrophile Sulfonierung.

13. Verfahren zur Herstellung von sulfonierten Liganden gemäß Anspruch 8 oder 9 aus 2-Hydroxy-2'-phosphinobiphenylen durch Metallierungsreaktion und anschließenden Quench mit einem Sulfonierungsreagenz.

## Claims

1. Process for preparing aryl- or heteroarylamines, aryl or heteroaryl ethers or aryl or heteroaryl thioethers (III) by cross-coupling primary or secondary amines, alcohols or thioalcohols (II) with substituted aryl or heteroaryl compounds (I), in the presence of a Brønsted base and of a catalyst or precatalyst comprising
a.) a transition metal, a complex, a salt or a compound of this transition metal from the group of {V, Mn, Fe, Co, Ni, Rh, Pd, Ir, Pt} , and
b.) at least one sulphonated phosphine ligand
in a solvent or solvent mixture, according to scheme 1 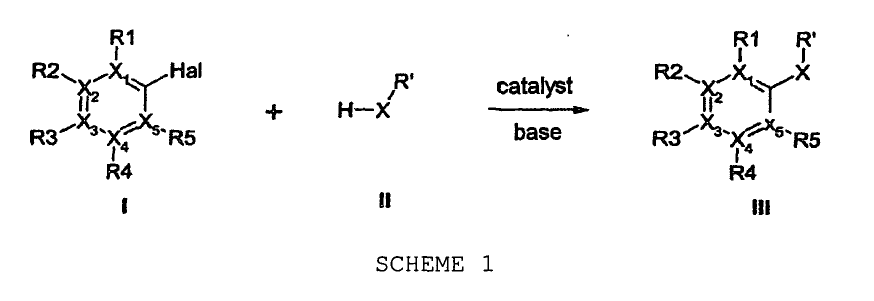 where Hal is fluorine, chlorine, bromine, iodine, alkoxy, trifluoromethanesulphonate, nonafluorotrimethylmethanesulphonate,
methanesulphonate, 4-toluenesulphonate, benzenesulphonate, 2-naphthalenesulphonate, 3-nitrobenzenesulphonate, 4-nitrobenzenesulphonate, 4-chlorobenzenesulphonate, 2,4,6-triisopropylbenzenesulphonate or any other sulphonate,
X is O, S or NR",
X₁₋₅ are each independently carbon, or XᵢRᵢ is nitrogen, or in each case two adjacent XᵢRᵢ bonded via a formal double bond together are 0 (furans), S (thiophenes), NH or NRᵢ (pyrroles),
the R₁₋₅ radicals are substituents from the group of {hydrogen, methyl, primary, secondary or tertiary, cyclic or acyclic alkyl radicals having from 2 to 20 carbon atoms, in which one or more hydrogen atoms are optionally replaced by fluorine or chlorine or bromine, substituted cyclic or acyclic alkyl groups, hydroxyl, alkoxy, amino, alkylamino, dialkylamino, arylamino, diarylamino, alkylarylamino, pentafluorosulphanyl, phenyl, substituted phenyl, heteroaryl, substituted heteroaryl, thio, alkylthio, arylthio, diarylphosphino, dialkylphosphino, alkylarylphosphino, optionally substituted aminocarbonyl, CO₂⁻, alkyl- or aryloxycarbonyl, hydroxyalkyl, alkoxyalkyl, fluorine or chlorine, nitro, cyano, aryl- or alkylsulphone, aryl- or alkylsulphonyl} or in each case two adjacent R₁₋₅ radicals together are an aromatic, heteroaromatic or aliphatic fused-on ring,
when X = O or S, R' may be identical or different radicals from the group of {hydrogen, methyl, linear, branched C₁-C₂₀ alkyl or cyclic alkyl, substituted or unsubstituted aryl or heteroaryl},
or X is NR", where R' and R" are each independently identical or different radicals from the group of {hydrogen, methyl, linear, branched C₁-C₂₀ alkyl or cyclic alkyl, substituted or unsubstituted aryl or heteroaryl} or together form a ring,
and wherein a sulphonated phosphine ligand of the structure 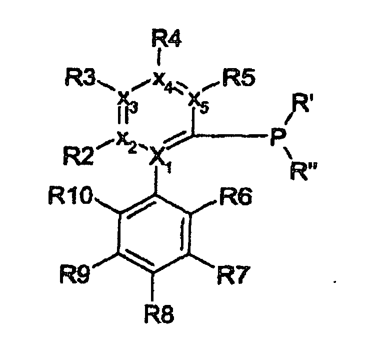 is used, where
X₁ is carbon or nitrogen,
X₂₋₅ are each independently carbon, or
XᵢRᵢ is nitrogen, or in each case two adjacent XᵢRᵢ bonded via a formal double bond, where i = 2, 3, 4, 5, together are 0 (furans), S (thiophenes), NH or NRᵢ (pyrroles),
the radicals
R₂₋₁₀ are each substituents from the group of {hydrogen, methyl, primary, secondary or tertiary, cyclic or acyclic alkyl radicals having from 2 to 20 carbon atoms, in which one or more hydrogen atoms are optionally replaced by fluorine or chlorine or bromine, substituted cyclic or acyclic alkyl groups, hydroxyl, alkoxy, amino, alkylamino, dialkylamino, arylamino, diarylamino, alkylarylamino, phenyl, substituted phenyl, heteroaryl, substituted heteroaryl, thio, alkylthio, arylthio, diarylphosphino, dialkylphosphino, alkylarylphosphino, optionally substituted aminocarbonyl, CO₂⁻, alkyl- or aryloxycarbonyl, hydroxyalkyl, alkoxyalkyl, nitro, cyano, aryl- or alkylsulphone, aryl- or alkylsulphonyl},
where at least one radical represents a sulphonic acid or sulphonate group,
and where at least one radical in the suphonated ring is a free OH group,
or in each case two adjacent radicals
R₂₋₅ together are an aromatic, heteroaromatic or aliphatic fused-on ring,
R' and R" are each independently identical or different radicals from the group of {hydrogen, methyl, linear, branched or cyclic alkyl, phenyl} or together form a ring and are a bridging structural element from the group of {alkylene, branched alkylene, cyclic alkylene}, or are each independently one or two polycyclic radicals, for example norbornyl or adamantyl.

2. Process according to Claim 1, **characterized in that** sulphonated phosphine ligands which contain at least one sulphonic acid group or a metal sulphonate are used.

3. Process according to Claim 1 and/or 2, **characterized in that** the Brønsted base used is a hydroxide, alkoxide or amide of the alkali metals or alkaline earth metals or an alkali metal carbonate or phosphate or mixtures of these compounds.

4. Process according to at least one of the preceding claims, **characterized in that** from 1.0 to 3 equivalents of base based on the aryl or heteroaryl halide or aryl or heteroaryl sulphonate are used.

5. Process according to at least one of the preceding claims, **characterized in that** the solvents used are hydrocarbons, halogenated hydrocarbons, open-chain and cyclic ethers and diethers, oligo- and polyethers, tertiary amines, DMSO, NMP, DMF, DMAc, and substituted mono- or polyalcohols and optionally substituted aromatics, or a mixture of a plurality of these solvents.

6. Process according to at least one of the preceding claims, **characterized in that** the reaction is performed at a temperature in the range from 0 to 240°C.

7. Process according to at least one of the preceding claims, **characterized in that** the catalyst is used in a ratio relative to the reactant (I) in amounts of from 0.001 mol% to 100 mol%.

8. Sulphonated ligands of the structure 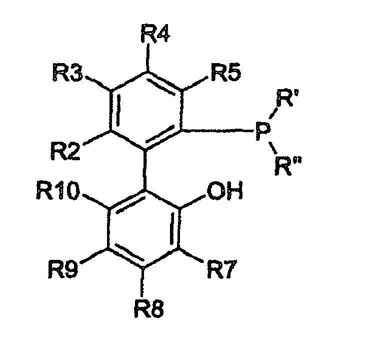 where the radicals
R₂₋₅ and R₇₋₁₀ are each substituents from the group of {hydrogen, methyl, primary, secondary or tertiary, cyclic or acyclic alkyl radicals having from 2 to 20 carbon atoms, in which one or more hydrogen atoms are optionally replaced by fluorine or chlorine or bromine, substituted cyclic or acyclic alkyl groups, hydroxyl, alkoxy, amino, alkylamino, dialkylamino, arylamino, diarylamino, alkylarylamino, phenyl, substituted phenyl, heteroaryl, substituted heteroaryl, thio, alkylthio, arylthio, diarylphosphino, dialkylphosphino, alkylarylphosphino, optionally substituted aminocarbonyl, CO₂⁻, alkyl- or aryloxycarbonyl, hydroxyalkyl, alkoxyalkyl, nitro, cyano, aryl- or alkylsulphone, aryl- or alkylsulphonyl}, where at least one radical represents a sulphonic acid or sulphonate group,
or in each case two adjacent radicals
R₂₋₅ together are an aromatic, heteroaromatic or aliphatic fused-on ring,
R' and R" are each independently identical or different radicals from the group of {hydrogen, methyl, linear, branched or cyclic alkyl, phenyl} or together form a ring and are a bridging structural element from the group of {alkylene, branched alkylene, cyclic alkylene}, or are each independently one or two polycyclic radicals, for example norbornyl or adamantyl.

9. Sulphonated ligands of the structure 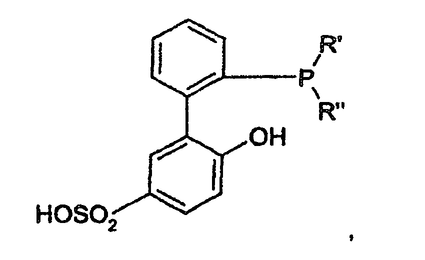 in which
R' and R" are each independently identical or different radicals from the group of {hydrogen, methyl, linear, branched or cyclic alkyl, phenyl} or together form a ring and are a bridging structural element from the group of {alkylene, branched alkylene, cyclic alkylene} or are each independently one or two polycyclic radicals, for example norbornyl or adamantyl.

10. Complexes, mixtures, salts or formulations comprising at least one ligand according to Claim 8 or 9 and at least one metal, metal complex, metal salt or metal compound from the group of {V, Mn, Fe, Co, Ni, Rh, Pd, Ir, Pt}.

11. Use of ligands according to Claim 8 or 9 for catalysts in organochemical catalysis.

12. Process for preparing sulphonated ligands according to Claim 8 or 9 from 2-hydroxy-2'-phosphinobiphenyls by electrophilic sulphonation.

13. Process for preparing sulphonated ligands according to Claim 8 or 9 from 2-hydroxy-2'-phosphinobiphenyls by metallation reaction and subsequent quenching with a sulphonation reagent.

## Revendications

1. Procédé de fabrication d'aryl- ou d'hétéroarylamines, -éthers ou -thioéthers (III) par couplage croisé d'amines, d'alcools ou de thioalcools (II) primaires ou secondaires avec des composés d'aryle ou d'hétéroaryle substitués (I), en présence d'une base de Brønsted et d'un catalyseur ou d'un précatalyseur, contenant :
a.) un métal de transition, un complexe, un sel ou un composé de ce métal de transition tiré du groupe {V, Mn, Fe, Co, Ni, Rh, Pd, Ir, Pt}, et
b.) au moins un ligand de phosphine sulfoné, dans un solvant ou un mélange de solvants selon le schéma 1 suivant : 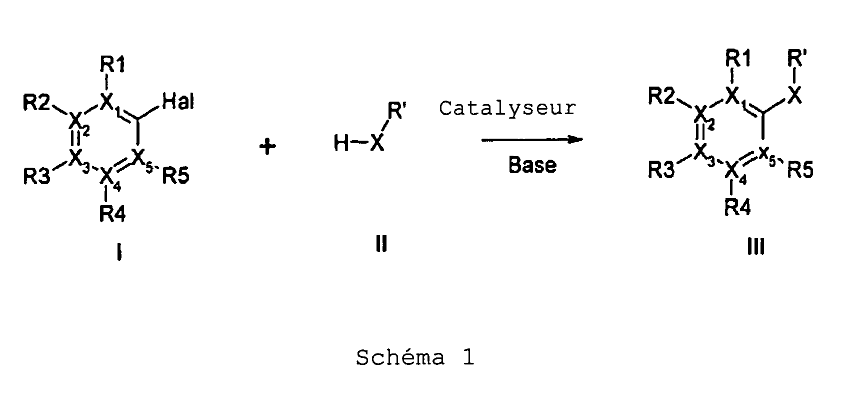
où le terme Hal représente le fluor, le chlore, le brome, l'iode, un groupement alcoxy, trifluorométhanesulfonate, nonafluorotriméthylméthanesulfonate, méthanesulfonate, 4-toluènesulfonate, benzènesulfonate, 2-naphtalènesulfonate, 3-nitrobenzènesulfonate, 4-nitrobenzènesulfonate, 4-chlorobenzènesulfonate, 2,4,6-triisopropylbenzènesulfonate ou un autre sulfonate quelconque,
X représente l'élément 0, S ou un groupement NR",
les entités X₁₋₅ représentent, indépendamment l'une de l'autre, un atome de carbone, le radical XᵢRᵢ représente l'azote ou, respectivement, deux radicaux XᵢRᵢ voisins, reliés sous forme d'une double liaison formelle, représentent conjointement l'élément O (furanes), S (thiophènes), un groupement NH ou NRᵢ (pyrroles),
les radicaux R₁₋₅ représentent des substituants choisis dans le groupe {hydrogène, méthyle, radicaux alkyle primaires, secondaires ou tertiaires, cycliques ou acycliques, ayant 2 à 20 atomes de carbone, dans lesquels, éventuellement, un ou plusieurs atomes d'hydrogène sont remplacés par des atomes de fluor, de chlore ou de brome, groupements alkyle substitués cycliques ou acycliques, hydroxyle, alcoxy, amino, alkylamino, dialkylamino, arylamino, diarylamino, alkylarylamino, pentafluorosulfuranyle, phényle, phényle substitué, hétéroaryle, hétéroaryle substitué, thio, alkylthio, arylthio, diarylphosphino, dialkylphosphino, alkylarylphosphino, aminocarbonyle éventuellement substitué, CO₂⁻, alkyl- ou aryloxycarbonyle, hydroxyalkyle, alkoxyalkyle, fluor ou chlore, nitro, cyano, aryl- ou alkylsulfone, aryl- ou alkylsulfonyle} ou, respectivement, deux radicaux R₁₋₅ voisins représentent conjointement un cycle condensé aromatique, hétéroaromatique ou aliphatique,
lorsque X est l'élément O ou S, R' peut représenter des radicaux identiques ou différents choisis dans le groupe {hydrogène, méthyle, alkyle en C₁-C₂₀ linéaire ou ramifié ou alkyle cyclique, aryle ou hétéroaryle substitué ou non substitué},
ou X représente NR", où R' et R" représentent, indépendamment l'un de l'autre, des radicaux identiques ou différents choisis dans le groupe {hydrogène, méthyle, alkyle en C₁-C₂₀ linéaire ou ramifié ou alkyle cyclique, aryle ou hétéroaryle substitué ou non substitué} ou forment conjointement un cycle,
et où un ligand de phosphine sulfoné de structure suivante : 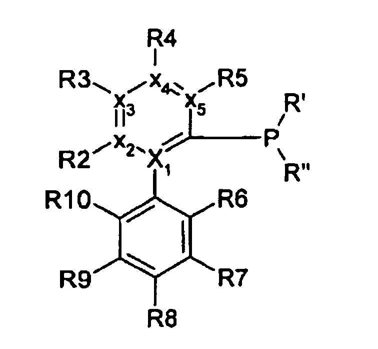 est utilisé,
où
X₁ représente un atome de carbone ou d'azote,
les entités X₂₋₅ représentent, indépendamment l'une de l'autre, un atome de carbone,
XᵢRᵢ représente un atome d'azote ou, respectivement, deux radicaux XᵢRᵢ voisins, reliés sous forme d'une double liaison formelle, avec i = 2, 3, 4, 5, représentent conjointement l'élément O (furanes), S (thiophènes), un groupement NH ou NRᵢ (pyrroles),
les radicaux
R₂₋₁₀ représentent des substituants choisis dans le groupe
{hydrogène, méthyle, radicaux alkyle primaires, secondaires ou tertiaires, cycliques ou acycliques, ayant 2 à 20 atomes de carbone, dans lesquels, éventuellement, un ou plusieurs atomes d'hydrogène sont remplacés par des atomes de fluor, de chlore ou de brome, groupements alkyle cycliques ou acycliques substitués, hydroxyle, alcoxy, amino, alkylamino, dialkylamino, arylamino, diarylamino, alkylarylamino, phényle, phényle substitué, hétéroaryle, hétéroaryle substitué, thio, alkylthio, arylthio, diarylphosphino, dialkylphosphino, alkylarylphosphino, aminocarbonyle éventuellement substitué, CO₂⁻, alkyl- ou aryloxycarbonyle, hydroxyalkyle, alcoxyalkyle, nitro, cyano, aryl- ou alkylsulfone, aryl- ou alkylsulfonyle},
au moins un radical représentant un groupement acide sulfonique ou sulfonate
et au moins un radical dans le cycle sulfoné représentant un groupement OH libre,
ou, respectivement, deux radicaux
R₂₋₅ voisins représentent conjointement un cycle condensé aromatique, hétéroaromatique ou aliphatique,
R' et R" représentent, indépendamment l'un de l'autre, des radicaux identiques ou différent choisis dans le groupe
{hydrogène, méthyle, alkyle linéaire, ramifié ou cyclique, phényle}
ou
forment conjointement un cycle et représentent un élément de structure ponté choisi dans le groupe {alkylène, alkylène ramifié, alkylène cyclique} ou représentent, indépendamment l'un de l'autre,
un ou deux radicaux polycycliques, tels qu'un groupement norbornyle ou adamantyle.

2. Procédé selon la revendication 1, **caractérisé en ce que** l'on utilise des ligands de phosphine sulfonés, qui contiennent au moins un groupement acide sulfonique ou un sulfonate de métal.

3. Procédé selon la revendication 1 et/ou 2, **caractérisé en ce que** l'on utilise, comme base de Brønsted, un hydroxyde, un alcoolate ou un amide des métaux alcalins ou alcalinoterreux ou un carbonate alcalin ou un phosphate alcalin ou des mélanges de ces composés.

4. Procédé selon au moins l'une quelconque des revendications précédentes, **caractérisé en ce que** l'on utilise 1,0 à 3 équivalents de base par rapport à l'halogénure d'aryle ou d'hétéroaryle ou par rapport à l'arylsulfonate ou à l'hétéroarylsulfonate.

5. Procédé selon au moins l'une quelconque des revendications précédentes, **caractérisé en ce que** l'on utilise, comme solvant, des hydrocarbures, des hydrocarbures halogénés, des éthers et des diéthers à chaîne ouverte ou cycliques, des oligo- et polyéthers, des amines tertiaires, le DMSO, la NMP, le DMF, le DMAc, ainsi que des alcools substitués une ou plusieurs fois et, éventuellement, des composés aromatiques substitués ou un mélange de plusieurs de ces solvants.

6. Procédé selon au moins l'une quelconque des revendications précédentes, **caractérisé en ce que** la réaction est effectuée à une température située dans la plage de 0 à 240 °C.

7. Procédé selon au moins l'une quelconque des revendications précédentes, **caractérisé en ce que** le catalyseur est utilisé, par rapport au réactif (I), en quantités de 0,001 % en mole à 100 % en mole.

8. Ligands sulfonés de structure suivante : 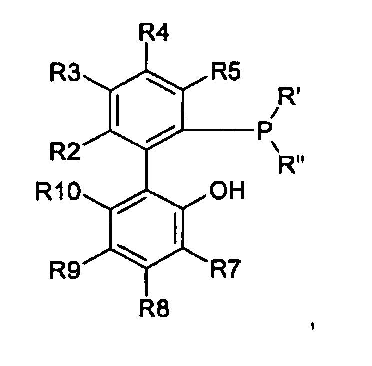 dans laquelle les radicaux
R₂₋₅ et R₇₋₁₀ représentent des substituants choisis dans le groupe
{hydrogène, méthyle, radicaux alkyle primaires, secondaires ou tertiaires, cycliques ou acycliques, ayant 2 à 20 atomes de carbone, dans lesquels, éventuellement, un ou plusieurs atomes d'hydrogène sont remplacés par des atomes de fluor, de chlore ou de brome, groupements alkyle cycliques ou acycliques substitués, hydroxyle, alcoxy, amino, alkylamino, dialkylamino, arylamino, diarylamino, alkylarylamino, phényle, phényle substitué, hétéroaryle, hétéroaryle substitué, thio, alkylthio, arylthio, diarylphosphino, dialkylphosphino, alkylarylphosphino, aminocarbonyle éventuellement substitué, CO₂⁻, alkyl- ou aryloxycarbonyle, hydroxyalkyle, alcoxyalkyle, nitro, cyano, aryl- ou alkylsulfone, aryl- ou alkylsulfonyle},
au moins un radical représentant un groupement acide sulfonique ou sulfonate
ou, respectivement, deux radicaux
R₂₋₅ voisins représentent conjointement un cycle condensé aromatique, hétéroaromatique ou aliphatique,
R' et R" représentent, indépendamment l'un de l'autre, des radicaux identiques ou différent choisis dans le groupe
{hydrogène, méthyle, alkyle linéaire, ramifié ou cyclique, phényle}
ou
forment conjointement un cycle et représentent un élément de structure ponté choisi dans le groupe {alkylène, alkylène ramifié, alkylène cyclique} ou représentent, indépendamment l'un de l'autre,
un ou deux radicaux polycycliques, tels qu'un groupement norbornyle ou adamantyle.

9. Ligands sulfonés de structure suivante : 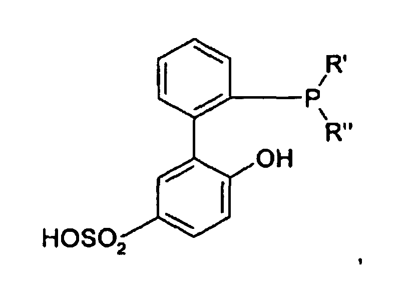 dans laquelle
R' et R" représentent, indépendamment l'un de l'autre, des radicaux identiques ou différent choisis dans le groupe
{hydrogène, méthyle, alkyle linéaire, ramifié ou cyclique, phényle}
ou
forment conjointement un cycle et représentent un élément de structure ponté choisi dans le groupe {alkylène, alkylène ramifié, alkylène cyclique} ou représentent, indépendamment l'un de l'autre,
un ou deux radicaux polycycliques, tels qu'un groupement norbornyle ou adamantyle.

10. Complexes, mélanges, sels ou formulations contenant au moins un ligand selon la revendication 8 ou 9 et au moins un métal, un complexe, un sel ou un composé d'un métal tiré du groupe {V, Mn, Fe, Co, Ni, Rh, Pd, Ir, Pt}.

11. Utilisation de ligands selon la revendication 8 ou 9 pour des catalyseurs dans le cadre de la catalyse organo-chimique.

12. Procédé de fabrication de ligands sulfonés selon la revendication 8 ou 9, à partir du 2-hydroxy-2'-phosphinobiphénylène par sulfonation électrophile.

13. Procédé de fabrication de ligands sulfonés selon la revendication 8 ou 9, à partir du 2-hydroxy-2'-phosphinobiphénylène par une réaction de métallation, puis par blocage en utilisant un réactif de sulfonation.
